**Europäisches Patentamt**

**European Patent Office**

**Office européen des brevets**

(19)

(11) Veröffentlichungsnummer: **0 214 368**
**B1**

(12) **EUROPÄISCHE PATENTSCHRIFT**

(45) Veröffentlichungstag der Patentschrift:
12.07.89

(21) Anmeldenummer: **86105607.5**

(22) Anmeldetag: **23.04.86**

(51) Int. Cl.⁴: **A61F 13/00**, A61F 7/02,
A61N 1/42, B32B 33/00

(54) **Mittel zur Steigerung und Stabilisierung der Hauttemperatur.**

(30) Priorität: **07.09.85 DE 8525561 U**

(43) Veröffentlichungstag der Anmeldung:
**18.03.87 Patentblatt 87/12**

(45) Bekanntmachung des Hinweises auf die Patenterteilung:
**12.07.89 Patentblatt 89/28**

(84) Benannte Vertragsstaaten:
**AT BE CH DE FR GB IT LI NL SE**

(56) Entgegenhaltungen:
**EP-A- 0 076 354**
**EP-A- 0 136 393**
**AU-B- 424 257**
**DE-A- 2 362 908**
**DE-A- 2 722 273**

(73) Patentinhaber: **LACOTHERM AG, Kronenstrasse 191,
CH-9427 Wolfhalden(CH)**

(72) Erfinder: **Latzke, Arno W., Mühltobel 946,
CH-9429 Zelg-Wolfhalden(CH)**
Erfinder: **Latzke, Ralf, Mühltobel 946,
CH-9429 Zelg-Wolfhalden(CH)**
Erfinder: **Latzke, Gert, Kronenstrasse 191,
CH-9427 Wolfhalden(CH)**

(74) Vertreter: **Werner, Hans-Karsten, Dr. et al,
Deichmannhaus am Hauptbahnhof, D-5000 Köln 1(DE)**

**Beschreibung**

Gegenstand der vorliegenden Erfindung ist ein Mittel zur Steigerung und Stabilisierung der Hauttemperatur, welches anwendbar ist bei Muskel- und Gelenkschmerzen, oberflächigen Blutergüssen, Schwellungen, Zerrungen, Prellungen und Tennisarm.

Aus der US-PS 4,489,711, bzw. EP-A 136 393, sind Magnetpflaster bekannt, welche aus flexiblen, hautverträglichen, permanent magnetisierbaren und magnetisierten Kunststoff-Folien bestehen, welche selbstklebend sind oder mit Hilfe von hautverträglichen, selbstklebenden Pflastern auf der Haut befestigt werden können. Vorzugsweise sind diese Pflaster abwechselnd positiv und negativ magnetisiert, wobei die positiven und negativen Pole einen Abstand von 3 bis 15 mm, vorzugsweise 4 bis 10 mm aufweisen. Diese Magnetpflaster bewirken unter anderem eine Steigerung der Durchblutung und eine gewisse Erhöhung der Temperatur in unmittelbarer Nähe der Magnetfelder.

Die Erfindung hat sich die Aufgabe gestellt, die Wirksamkeit zu steigern und die Anwendbarkeit zu erleichtern und angenehmer zu machen. Es wurde jetzt gefunden, daß diese Aufgabe gelöst werden kann durch ein Mittel bestehend aus in der folgenden Reihenfolge angeordneten Schichten

a) einer ersten 0,8 bis 5 mm starken geschlossenporigen Schaumstoffschicht (1),

b) einer flexiblen, wärmeleitenden Metallschicht (2),

c) einer zweiten 0,8 bis 5 mm starken geschlossenporigen Schaumstoffschicht (3) und

d) einer oder mehreren in die zweite Schaumstoffschicht (3) eingelagerten streifenförmig abwechselnd positiv und negativ dauerhaft magnetisierten Kunststoff-Folien (4), oder einer oder mehreren orientiert angeordnete, dauerhaft axial magnetisierte Kunststoff-Folien oder Ferritkerne sowie gegebenenfalls

e) einer selbstklebenden porösen Textilschicht (5),

wobei, diese Mittel entweder mit Hilfe von hautverträglichen, selbstklebenden Pflastern oder durch Klettverschlüsse und Binden auf der Haut dauerhaft aufbringbar sind.

Es wurde nähmlich gefunden, daß die durch die Magnetfelder erzeugte Wärme durch die übrigen Bestandteile des neuen Mittels in ausgezeichneter Weise gespeichert, umverteilt und damit insgesamt gesteigert wird. Gleichzeitig führt das Mittel zu einer Stabilisierung der erhöhten Hauttemperatur.

Da eine Reihe von Schmerzen, Verkrampfungen und krampfhaften Störungen auf einer ungenügenden oder ungleichmäßigen Durchblutung der Außenhaut und der darunter liegenden Körperteile beruhen, kann in vielen Fällen durch gleichmäßige Einwirkung von Wärme Besserung oder sogar Heilung erzielt werden. Die Behandlung mit Infrarotstrahlen, Wärmflaschen, Heizkissen, heißen Packungen und Umschlägen ist stets mit erheblichem Aufwand verbunden und daher nicht immer und ohne weiteres durchführbar.

Die Kombination aus der wärmeisolierenden Schaumstoffschicht (1), der gut wärmeleitende, flexiblen Metallschicht (2) und der weiteren geschlossenporigen Schaumstoffschicht (3) bewirkt für sich bereits eine ausgezeichnete Speicherung und Umverteilung der Körperwärme.

Die Kombintion aus zwei Schaumstoffschichten und einer Metallschicht führt weiterhin zu einer mechanischen Stabilisierung, wodurch das erfindungsgemäße Mittel auch strapazierfähiger wird und nicht mehr so leicht reißt und bricht wie Schaumstoffschichten alleine oder die bisher handelsüblichen magnetisierten Kunststoff-Folien. Die erfindungsgemäßen Mittel sind nicht nur wärmeisolierend, sondern wirken obendrein als Dampfsperren. Es kommt somit unterhalb der erfindungsgemäßen Mittel auch zu einem gewissen Feuchtigkeitsstau. Bekanntlich ist jedoch feuchte Wärme therapeutisch besonders wirkungsvoll. Die Isolierwirkung der Schichten (1), (2) und (3) zusammen ist wesentlich höher als die herkömmlicher Isolierstoffe. Dieser Effekte beruht darauf, daß ein guter Wärmeleiter mit guten Wärmeisolatoren kombiniert wird. Diese Kombination von gegensätzlich wirksamen Schichten ist bisher vom Fachmann nicht in Erwägung gezogen worden, da zur Erzielung einer guten Wärmeisolierung nur Wärmeisolatoren verwendet wurden und dabei die Verwendung von gut wärmeleitfähigen Werkstoffen vermieden wurde. Die erfindungsgemäße Kombination bewirkt somit nicht nur eine gute Wärmespeicherung, sondern gleichzeitig eine gute flächenmäßige Verteilung der Wärme. Dabei wird die Hauttemperatur zusätzlich durch die Magnetfelder gesteigert, so daß insgesamt bisher nicht beobachtete Temperatursteigerungen über längere Zeiträume bewirkt werden können.

Da optimale magnetische Wirkungen beobachtet werden, wenn die Kunststoff-Folien in Abständen von 4 bis 10 mm abwechselnd positiv und negativ magnetisiert sind und eine magnetische Feldstärke von 100 bis 2000 Gauss aufweisen, werden vorzugsweise solche Kunststoff-Folien eingesetzt.

Optimale Ergebnisse werden offensichtlich bei Abständen von 4 bis 6 mm und Feldstärke zwischen 250 und 1200 Gauss erzielt. Höhere Feldstärken sind mit den bisher handelsüblichen Folien nur dann erreichbar, wenn die Foliendicke entsprechend erhöht wird. Dies führt jedoch zu einer Versteifung des Gesamtmittels. Diese Versteifung wird zwar im tiermedizinischen Bereich, insbesondere bei Pferden, akzeptiert, nicht jedoch im Humanbereich.

Besonders gute Verträglichkeit und Wirksamkeit wird beobachtet, wenn die der Haut zugewandte Seite der Kunststoff-Folie mit einer Goldfolie überzogen ist. Die Ergebnisse deuten darauf hin, daß diese Goldfolie zu einer zusätzlichen Wärmereflektion führt und den Effekt des erfindungsgemäßen Mittels weiter steigert.

Als Metallschicht (2) hat sich insbesondere Aluminiumfolie bewährt, die preiswert, stabil und leicht verarbeitbar ist.

Die Kunststoff-Folien (4) werden in die Schaumstoffschicht (3) eingelagert, indem entsprechende Teile dieser Schaumstoffschicht unter Wärme zusammengepreßt werden und dadurch permanent entsprechende Aussparungen für die Kunststoff-Folie bilden. Prinzipiell ist es möglich, die Kunststoff-Folie mit dem komprimierten Schaumstoff zu verkleben. Besonders bevorzugt ist jedoch eine Ausführungsform, bei der eine selbstklebende poröse Textilschicht (5) vorhanden ist, welche die magnetisierten Kunststoff-Folien in den Aussparungen der Schaumstoffschicht (3) hält.

Die Befestigung der erfindungsgemäßen Mittel auf der Haut, bzw. bei Tieren auf dem Fell, erfolgt in an sich bekannter Weise entweder mittels hautverträglicher selbstklebender Pflaster oder durch Klettverschlüsse und Binden.

Insbesondere bei Verwendung von Klettverschlüssen und Binden ist es möglich, sowohl die erfindungsgemäßen Mittel wie die Binden separat zu reinigen und in hygienischer Form wiederzuverwenden. Sie können sogar bis 60°C mit Seife oder Waschmitteln gewaschen werden.

Die geschlossenporigen Schaumstoffschichten sind geruchsneutral und bilden auch nach längerer Benutzung keinen Nährboden für Pilze etc. Die erfindungsgemäßen Mittel entsprechen somit in jeder Hinsicht den heutigen hygienischen Anforderungen. Sofern sie mit selbstklebenden Pflastern befestigt sind, können sie auch beim Baden und Duschen weitergetragen werden. Mittel, die mittels eines Klettverschlusses und einer Binde auf der Haut befestigt wurden, lassen sich demgegenüber leicht entfernen und anschließend wieder applizieren.

In Fällen, in denen eine besondere Empfindlichkeit gegen Feuchtigkeit und eigenen Schweiß besteht, kann die Unterseite der Schaumstoffschicht (3) genoppt werden, so daß durch diese Rillen die Feuchtigkeit besser entweichen kann. Ein ähnlicher Effekt wird erzielt, wenn das gesamte Mittel in gewissen Abständen perforiert wird. Der dabei eintretende Wärmeverlust ist gering im Verhältnis zu dem gewünschten Effekt der Entfernung der Feuchtigkeit.

In den nachfolgenden Beispielen und Figuren sind typische Ausführungsformen der erfindungsgemäßen Mittel dargestellt. In sämtlichen Figuren bedeutet

1: eine geschlossenporige Schaumstoffschicht, die vorzugsweise aus Polyethylen ist,
2: eine flexible, wärmeleitende Metallschicht, die vorzugsweise in Form einer Aluminiumfolie vorliegt,
3: eine weitere geschlossenporige Schaumstoffschicht, die ebenfalls vorzugsweise aus Polyethylen besteht,
4: streifenförmig abwechselnd positiv und negativ magnetisierte Kunststoff-Folien,
5: eine selbstklebende poröse Textilschicht,
6: ein selbstklebendes Pflaster.

Figur 1 stellt die Aufsicht auf ein erfindungsgemäßes Mittel dar, welches in der Mitte einen kreisrunden und links und rechts daneben zwei etwa rechteckige magnetisierte Kunststoff-Folien enthält. Die Kunststoff-Folien sind entweder in Form von parallelen geraden Streifen oder in Form von konzentrischen Ringen magnetisiert (zirkularmagnetisiert).

Figur 2 zeigt einen Längsschnitt durch das Mittel gemäß Figur 1 auf der Linie A-B.

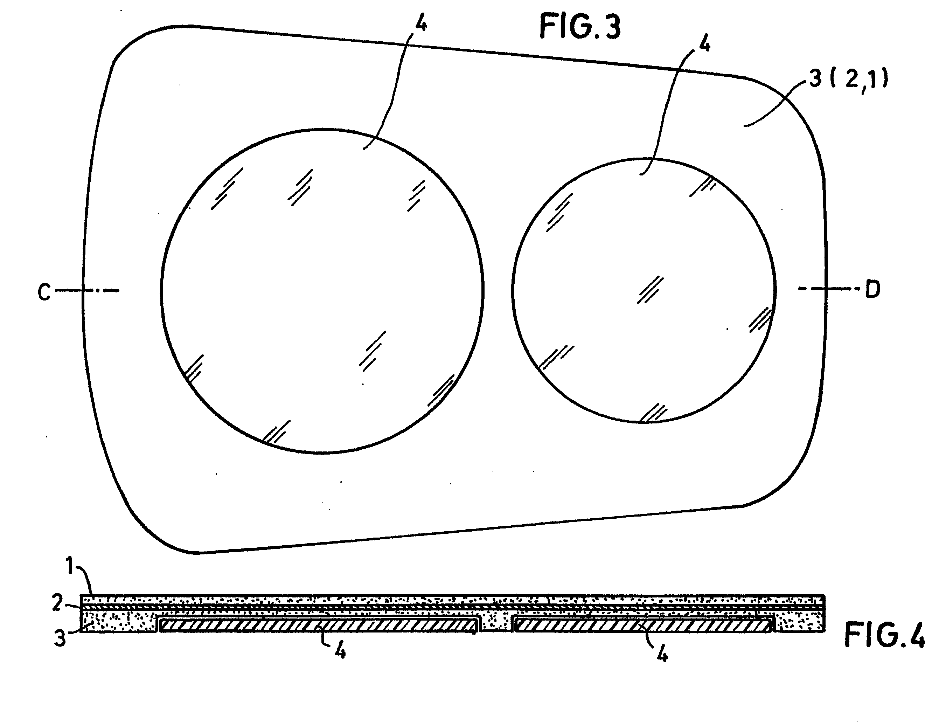

Figur 3 und 4 stellen ein erfindungsgemäßes Mittel für Veterinärzwecke, nämlich für Pferde, dar, wobei diese Mittel vorzugsweise mit Hilfe eines Klettverschlusses und einer Binde auf dem Fell des Pferdes angebracht werden können.

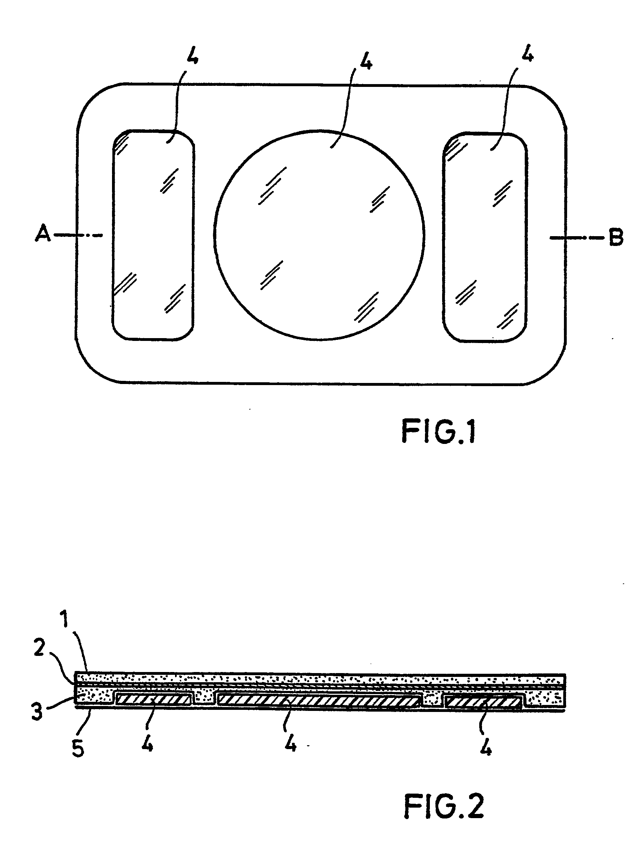

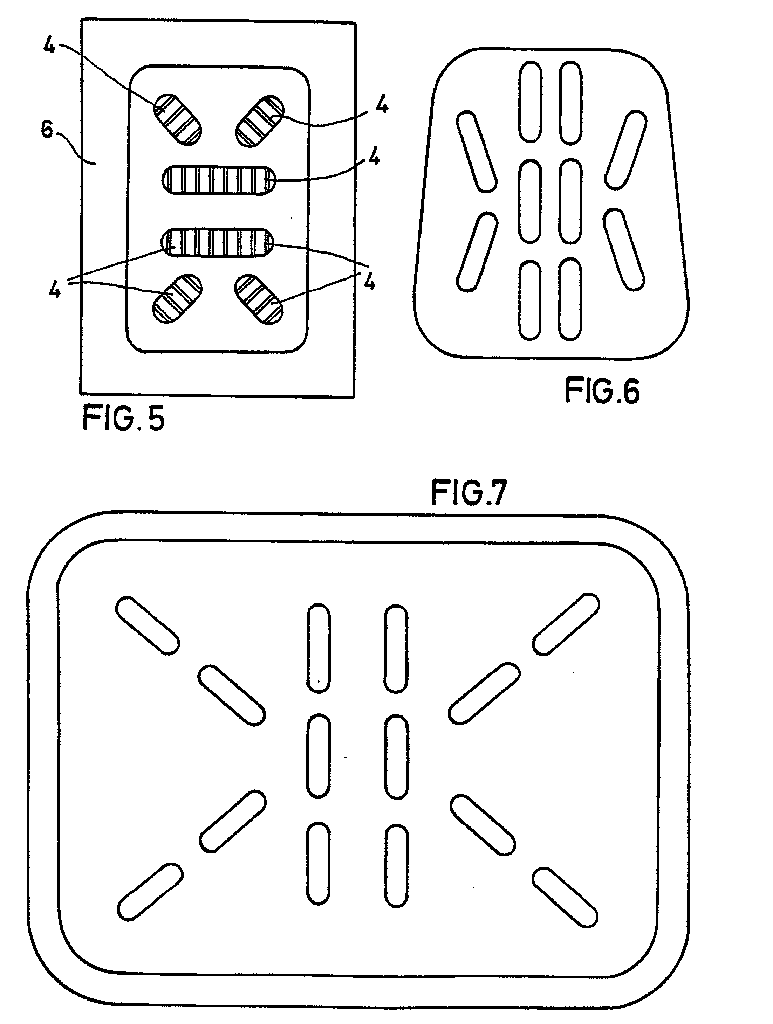

Figur 5 zeigt die Aufsicht einer weiteren Ausführungsform eines Mittels analog Figur 1 mit insgesamt sechs länglichen, abgerundeten Kunststoff-Folien, die quer mit parallelen geraden Streifen magnetisiert sind. Die Figur zeigt die Befestigung mittels eines selbstklebenden Pflasters, das rundherum über das Mittel herausragt und somit auf der Haut aufgeklebt werden kann. Die Schaumstoffschicht 1 trägt zusätzlich eine aufgeklebte oder auflaminierte Schicht aus einem schlingenförmig gewebten Nylongewebe (Borschednylon), welcher eine gute Haftung von Klettbändern einerseits und selbstklebenden Pflastern andererseits gewährleistet.

Figur 6 zeigt eine weitere Ausführungsform des erfindungsgemäßen Mittels für Veterinärzwecke mit insgesamt 10 länglichen, abgerundeten Kunststoff-Folien.

Figur 7 zeigt verkleinert eine kissenförmige Ausführungsform mit insgesamt 14 länglichen, abgerundeten Kunststoff-Folien. Die Rückseite des Kissens ist vorzugsweise genoppt. Die Kanten sind mit Textilband überzogen. Diese Ausführungsform kann insbesondere im Sitzen und im Liegen auf Stühlen, Autositzen und im Bett angewendet werden und muß nicht durch Hilfsmittel wie selbstklebende Pflaster, Klettverschlüsse oder Binden auf der Haut dauerhaft befestigt sein.

Prinzipiell ist es möglich, die streifenförmig abwechselnd positiv und negativ dauerhaft magnetisierten Kunststoff-Folien (4) ganz oder teilweise zu ersetzen durch orientiert angeordnete, dauerhaft axial mangetisierte Kunststoff-Folien oder orientiert angeordnete, dauerhaft magnetisierte Ferritkerne, sofern hierdurch ausreichend starke, in die Haut tief genug eindringende Magnetfelder entstehen, die eine deutliche Aktivierung des Blutes und des Stoffwechsels bewirken.

Bevorzugt werden jedoch die nachgewiesenermaßen wirksamen streifenförmig abwechselnd positiv und negativ dauerhaft magnetisierten Kunststoff-Folien mit parallelen geraden Streifen oder konzentrischen Ringen mit Abständen von 3 bis 15 mm, vorzugsweise 4 bis 10 mm.

Die Schaumstoffschichten (1) und (3) bestehen beispielsweise aus geschlossenporigem Polyethylenschaum von 3 mm Stärke und einem Raumgewicht von 30 kg/m³. Die Aluminiumfolie weist eine Stärke von 30 µm auf. Für die Pferdefolie wird vorzugsweise ein 5 mm starker Polyethylenweichschaum mit einem Raumgewicht von 50 kg/m³ verwendet. Als selbstklebende poröse Textilschicht (5) kommen insbesonder die Produkte "Curafix" der Firma Lohmann und "Fixomoll-Stretch" der Beiersdorf AG in Frage.

Ein weiterer Vorteil der neuen Mittel besteht darin, daß sie auch als längere Binden oder als größere Flächen ausgestaltet werden können, die dann auch größere Flächen der Haut abdecken können, ohne so steif und störend empfunden zu werden wie herkömmliche Magnetpflaster entsprechender großer Dimensionen. Ein weiterer Vorteil besteht darin, daß die erfindungsgemäßen Mittel wesentlich preiswerter sind als die herkömmlichen Magnetpflaster, da wesentlich weniger der relativ teuren Magnetfolie benötigt wird, um eine entsprechende Hautfläche abzudecken.

**Patentansprüche**

1. Mittel zur Steigerung und Stabilisierung der Hauttemperatur bestehend aus in der folgenden Reihenfolge angeordneten Schichten:
a) einer ersten 0,8 bis 5 mm starken geschlossenporigen Schaumstoffschicht (1),
b) einer flexiblen, wärmeleitenden Metallschicht (2),
c) einer zweiten 0,8 bis 5 mm starken geschlossenporigen Schaumstoffschicht (3) und
d) einer oder mehreren in die zweite Schaumstoffschicht (3) eingelagerten streifenförmig abwechselnd positiv und negativ dauerhaft magnetisierten Kunststoff-Folien (4), oder einer oder mehreren orientiert angeordnete, dauerhaft axial magnetisierte Kunststoff-Folien oder Ferritkerne, sowie gegebenenfalls
e) einer selbstklebenden porösen Textilschicht (5), wobei diese Mittel entweder mit Hilfe von hautverträglichen, selbstklebenden Pflastern oder durch Klettverschlüsse und Binden auf der Haut dauerhaft aufbringbar sind.

2. Mittel gemäß Anspruch 1, dadurch gekennzeichnet, daß die Kunststoff-Folien (4) in Abständen von 4 bis 10 mm abwechselnd positiv und negativ magnetisiert sind und eine magnetische Feldstärke von 100 bis 2000 Gauss aufweisen.

3. Mittel gemäß Anspruch 1 oder 2, dadurch gekennzeichnet, daß die der Haut zugewandte Seite der Kunststoff-Folien (4) mit einer Goldfolie überzogen ist.

4. Mittel gemäß einem der Ansprüche 1 bis 3, dadurch gekennzeichnet, daß die Metallschicht (2) eine Aluminiumfolie ist.

5. Mittel gemäß einem der Ansprüche 1 bis 4, dadurch gekennzeichnet, daß sie als Kissen ausgebildet sind.

**Claims**

1. Means for increasing and stabilizing the skin temperature which consist of layers positioned in sequence as follows:
(a) a first foam layer (1) comprising closed pores and being from 0.8 to 5 mm in thickness;
(b) a flexible heat-conducting metal layer (2);
(c) a second foam layer (1) comprising closed pores and being from 0.8 to 5 mm in thickness; and
(d) one or more strips made of alternatingly positively and negatively permanently magnetized films of synthetic materials (4), or one or more sheets of synthetic materials or ferrite cores having been permanently axially magnetized and orientatedly arranged, embedded in the second foam layer (3); as well as optionally

(e) a self-adhesive porous textile layer (5), said means being durably attachable to the skin either by using skin-compatible self-adhesive plasters or by means of Velcro strip fasteners and bandages.

2. Means according to claim 1, characterized in that the synthetic films (4) have been alternatingly positively and negatively magnetized at distances from 4 to 10 mm and have a magnetic field strength of from 100 to 2,000 Gauss.

3. Means according to claims 1 or 2, characterized in that the surface facing the skin of the synthetic films (4) is coated with a gold foil.

4. Means according to any one of claims 1 to 3, characterized in that the metal layer (2) is an aluminum foil.

5. Means according to any one of claims 1 to 4, characterized in that they are in the shape of a pad.

**Revendications**

1. Agents de traitement pour élever et stabiliser la température de la peau, constitués par des couches disposées dans l'ordre de succession suivant:
a) une première couche (1) de matière mousse à pores fermés, d'épaisseur 0,8 à 5 mm,
b) une couche métallique souple (2) thermoconductrice,
c) une deuxième couche (3) de matière mousse à pores fermés, d'épaisseur 0,8 à 5 mm et,
d) une ou plusieurs feuilles (4) de matière synthétique magnétisée de façon permanente, alternativement positivement et négativement par bandes incorporées dans la deuxième couche (3) de matière mousse, ou bien une ou plusieurs feuilles de matière synthétique ou noyaux de ferrite, magnétisées axialement de façon permanente et disposées avec orientation, ainsi qu'éventuellement
e) une couche textile poreuse autocollante (5), ces agents de traitement pouvant être appliqués, soit à l'aide d'emplâtres autocollants tolérés par la peau ou de rubans d'attache à autoaccrochage, sur la peau de manière permanente.

2. Agents de traitement selon la revendication 1, caractérisés en ce que les feuilles (4) de matière synthétique sont magnétisées alternativement positivement et négativement à des écartements de 4 à 10 mm et présentent une intensité de champ magnétique de 100 à 2000 gauss.

3. Agents de traitement selon la revendication 1 ou 2, caractérisés en ce que la face, tournée vers la peau, des feuilles (4) de matière synthétique est recouverte d'une feuille d'or.

4. Agents de traitement selon l'une des revendications 1 à 3, caractérisés en ce que la couche métallique (2) est une feuille d'aluminium.

5. Agents de traitement selon l'une des revendications 1 à 4, caractérisés en ce qu'ils sont constitués comme des coussins.

FIG.1

FIG.2

FIG.3

4

4

3 ( 2,1)

C

D

1

2

3

4

4

FIG.4

EP 0 214 368 B1

FIG.5

FIG.6

FIG.7